# EUROPÄISCHE PATENTSCHRIFT

(11) **EP 2 263 587 B1**
(45) Veröffentlichungstag und Bekanntmachung des Hinweises auf die Patenterteilung: **14.12.2016**
(21) Anmeldenummer: 10006191.0
(22) Anmeldetag: 15.06.2010
(51) Int. Cl.: A61B 18/14, A61B 18/12

(54) **Medizinisches Koagulationsinstrument**
Medical coagulation instrument
Instrument de coagulation médical

(30) Priorität: 16.06.2009 DE 102009025405
(43) Veröffentlichungstag der Anmeldung: 22.12.2010
(73) Patentinhaber: Karl Storz GmbH & Co. KG, 78532 Tuttlingen (DE)
(72) Erfinder: Bacher, Uwe, 78532 Tuttlingen (DE); Hermle, Rainer, 78559 Gosheim (DE)

(56) Entgegenhaltungen:
- US-A- 5 718 701
- US-A1- 2002 111 623
- US-A1- 2003 097 126
- US-A1- 2005 015 085
- US-A1- 2005 119 650

## Beschreibung

Die Erfindung betrifft ein medizinisches Koagulationsinstrument mit einem Schaft sowie mit mindestens einer das distale Ende des Schaftes überragen Elektrodenspitze.

Derartige medizinische Koagulationsinstrumente werden insbesondere verwendet, um bei der Operation über die mindestens eine mit Strom beaufschlagte Elektrodenspitze Gefäße zu verschließen.

Bei den medizinischen Koagulationsinstrumenten ist zu unterscheiden zwischen den sogenannten monopolaren Koagulationsinstrumenten und den bipolaren Koagulationsinstrumenten. Bei der sogenannten monopolaren Koagulation weist das Koagulationsinstrument eine mit Strom beaufschlagte Elektrodenspitze auf, über die der Strom unter Hitzeentwicklung an der kleinen Kontaktfläche der Elektrodenspitze mit dem Patienten in das Gewebe eintritt. Die Ableitung des Stroms erfolgt über eine Neutralelektrode, auf der der Patient großflächig aufliegt. Die großflächige Auflage des Patienten auf der Neutralelektrode verhindert eine Hitzentwicklung an der Stromaustrittsstelle.

Bei der bipolaren Koagulation weist das Koagulationsinstrument zwei gegeneinander elektrisch isolierte Elektrodenspitzen auf, die beide mit Strom beaufschlagt werden, so dass die Energie unter lokaler Hitzeentwicklung direkt von einer Elektrodenspitze zur anderen Elektrodenspitze übergeht.

Ein gattungsgemäßes, als bipolares Koagulationsinstrument ausgebildetes medizinisches Koagulationsinstrument ist beispielsweise aus der US 2008/0086121 A1 bekannt.

Nachteilig bei den bekannten Koagulationsinstrumenten ist, dass es beim Einführen des Instrumentes in das Operationsgebiet durch die mindestens eine über das distale Ende des Instrumentenschaftes vorstehenden Elektrode zu Verletzungen des Patienten kommen kann. Darüber hinaus bietet diese mindestens eine Elektrodenspitze nur eine geringe distale Koagulationsfläche und ist aufgrund ihrer konstruktiven Ausgestaltung instabil.

Weiterhin ist die US 5,718,701 A bekannt, die Elektroden offenbart, die so ausgebildet sind, dass sie einen sich aufweitenden distalen Bereich und einen relativ schmaleren proximalen Ansatzbereich aufweisen.

Nachteilig an diesen bekannten Instrumenten ist, dass die distal aufgeweiteten Elektrodenspitzen schwierig zu fertigen sind.

Davon ausgehend liegt der Erfindung die **Aufgabe** zugrunde, ein bipolares Koagulationsinstrument der eingangs genannten Art so auszugestalten, dass dessen Elektroden bei einfachem Aufbau eine atraumatische und sichere Handhabung gewährleisten.

Die **Lösung** dieser Aufgabenstellung ist erfindungsgemäß nach Anspruch 1 dadurch gekennzeichnet, dass die mindestens eine Elektrodenspitze sich nach distal aufweitend ausgebildet ist. Diese Aufweitung kann dabei sowohl stetig als auch stufenförmig in Achsrichtung des Koagulationsinstruments nach distal sein. Bei einer stufenförmigen Aufweitung ist jedoch darauf zu achten, dass diese Aufweitung in einer Position und Art ausgeführt ist, die bei der Anwendung bei einer Operation atraumatisch ist.

Ferner ist die Erfindung dadurch gekennzeichnet, dass zur Vereinfachung der Herstellung des Koagulationsinstruments an einer Elektrodenspitze außen ein Plättchen aufgebracht, insbesondere aufgeschweißt, wird. Dabei umfasst die Erfindung alle Arten, wie die Plättchen an die jeweilige Elektrodenspitze gefügt sind, und auch alle Kombinationen verschiedener Fügearten der beiden Plättchen.

Dieses Plättchen hat vorzugsweise im Wesentlichen die Form und insbesondere die Breite der Elektrodenspitze, so dass der Gesamtaufbau Elektrodenspitze und daran aufgebrachtes Plättchen quasi eine verdickte Elektrodenspitze bilden.

Das distale Ende des Plättchens reicht dabei bis zur distalen Endfläche der Elektrode beziehungsweise der Elektrodenspitze.

Das Plättchen ist dabei insbesondere ein Rohrsegment, insbesondere jedoch von der Form und Wölbung der Elektrodenspitze.

Das Plättchen kann dabei die Materialstärke der Elektrodenspitze, aber auch eine geringere oder auch größere Materialstärke aufweisen.

Durch die erfindungsgemäße Ausbildung der Elektrodenspitzen als sich nach distal aufweitende Elektroden ist es möglich, die Stabilität und Standfestigkeit der Elektroden zu erhöhen und gleichzeitig aufgrund der distalen Verbreiterung der Elektroden die Gefahr von Verletzungen des Patienten zu verringern.

Bei der Ausgestaltung des medizinischen Koagulationsinstruments als bipolares Koagulationsinstrument wird mit der Erfindung vorgeschlagen, dass am distalen Ende des Schaftes zwei das distale Ende des Schaftes überragende, gegeneinander elektrisch isolierten Elektrodenspitzen angeordnet sind, die beide sich nach distal aufweitend ausgebildet sind.

Gemäß der Erfindung wird vorgeschlagen, dass die Elektroden sich nach distal verdickend ausgebildet sind, um die Standfestigkeit der Elektroden durch die massivere Ausbildung der Elektrodenspitzen zu erhöhen.

Gemäß einer Ausführungsform der Erfindung wird vorgeschlagen, dass die Elektroden sich nach distal konisch erweiternd ausgebildet sind. Die konische Ausbildung der Elektrodenspitzen stellt eine besonders leicht zu fertigende und sicher zu handhabende Ausgestaltungsform der Elektroden dar, die die erhöhte Standfestigkeit bei gleichzeitiger Benutzungssicherheit miteinander vereint.

Gemäß einer alternativen Ausführungsform der Erfindung wird vorgeschlagen, dass die Elektroden sich nach distal fächerförmig erweiternd ausgebildet sind. Auch die fächerförmige Aufweitung der Elektrodenspitzen führt zur einer höheren Eigenstabilität der Elektrodenspitzen bei gleichzeitiger Verringerung der Verletzungsgefahr.

Weiterhin wird mit der Erfindung vorgeschlagen, dass zur Verbesserung der atraumatischen Handhabung des Koagulationsinstruments die freien Enden der Elektroden abgerundet ausgebildet sind.

Gemäß Erfindung kann die Materialstärke des Plättchens konstant sein. Die Materialstärke des Plättchens kann sich aber auch in Achsrichtung des Koagulations-instruments ändern, insbesondere nach distal erweitern, insbesondere konisch. Ebenso kann sich die Materialstärke des Plättchens nach distal verringern, insbesondere konisch. Außer konisch kann die Änderung der Materialstärke des Plättchens auch in jeder anderen Art ausgeführt sein, beispielsweise konvex oder konkav, stetig oder unstetig.

In derselben Weise kann sich die Materialstärke auch über den tangentialen Verlauf der Elektrodenspitze verändern. Beispielsweise kann die Materialstärke zu den tangentialen Außenabschnitten des Plättchen hin abnehmen.

Das Plättchen ist dabei insbesondere ein Rohrsegment, insbesondere jedoch von der Form und Wölbung der Elektrodenspitze.

Das Plättchen kann dabei die Materialstärke der Elektrodenspitze, aber auch eine geringere oder auch größere Materialstärke aufweisen.

Gemäß Erfindung kann die Materialstärke des Plättchens konstant sein. Die Materialstärke des Plättchens kann sich aber auch in Achsrichtung des Koagulations-instruments ändern, insbesondere nach distal erweitern, insbesondere konisch. Ebenso kann sich die Materialstärke des Plättchens nach distal verringern, insbesondere konisch. Außer konisch kann die Änderung der Materialstärke des Plättchens auch in jeder anderen Art ausgeführt sein, beispielsweise konvex oder konkav, stetig oder unstetig.

In derselben Weise kann sich die Materialstärke auch über den tangentialen Verlauf der Elektrodenspitze verändern. Beispielsweise kann die Materialstärke zu den tangentialen Außenabschnitten des Plättchen hin abnehmen.

Um die Verletzungsgefahr durch die tangentialen Außenabschnitte der Elektrodenspitze noch weiter zu verringern, könnte auch das Plättchen an seinen tangentialen Außenabschnitten verbreitert sein
Wie die Materialstärke der Elektrodenspitzen kann sich gemäß der Erfindung auch die Materialstärke des Plättchens ändern. Beispielsweise kann sich die Materialstärke des Plättchens über seinen axialen Verlauf, insbesondere nach distal konisch vergrößern.

Die Materialstärke des Plättchens auch über den tangentialen Verlauf des Plättchens verändern. Beispielsweise kann die Materialstärke zu den tangentialen Außenabschnitten des Plättchen hin abnehmen oder gegebenenfalls auch zunehmen.

Erfindungsgemäß kann auf die Elektrodenspitze zusätzlich oder statt dem Plättchen auf der Außenseite der Elektrodenspitze an dem distalen Endbereich des freien Endes der Elektrode ein Plättchen auf die Innenseite der Elektrodenspitze aufgebracht, insbesondere aufgeschweißt, sein.

Dieses innere Plättchen hat vorzugsweise die Form und Breite der Elektrodenspitze.

Das innere Plättchen ist dabei insbesondere ein Rohrsegment.

Das innere Plättchen kann dabei die Materialstärke der Elektrodenspitze, aber auch eine geringere oder auch größere Materialstärke aufweisen.

Gemäß der Erfindung kann sich die Materialstärke des inneren Plättchens ändern. Beispielsweise kann sich die Materialstärke des inneren Plättchens über seinen axialen Verlauf, insbesondere nach distal konisch vergrößern.

Die Materialstärke des inneren Plättchens auch über den tangentialen Verlauf des Plättchens verändern. Beispielsweise kann die Materialstärke zu den tangentialen Außenabschnitten des inneren Plättchen hin abnehmen.

Das äußere und/oder das innere Plättchen können außer auf der einen der beiden Elektroden auch auf beiden aufgebracht, insbesondere ausgeschweißt sein und zwar in jeglicher Form, also beispielsweise an einer Elektrode das innere Plättchen, an der anderen das äußere, an beiden Elektroden innere oder äußere Plättchen oder an einer Elektrode nur das innere oder äußere Plättchen und an der anderen beide Plättchen. Die jeweiligen Plättchen können dabei jeweils unabhängig jede der dargestellten Materialstärken bzw. Materialstärkenänderungen aufweisen.

Weitere Merkmale und Vorteile der Erfindung ergeben sich anhand der zugehörigen Zeichnungen, in denen ein Ausführungsbeispiel eines erfindungsgemäßen medizinischen Koagulationsinstruments nur beispielhaft dargestellt ist, ohne die Erfindung auf dieses Ausführungsbeispiel zu beschränken. In der Zeichnung zeigt:
- Fig. 1: eine perspektivische Seitenansicht eines erfindungsgemäßen medizinischen Koagulationsinstruments und
- Fig. 2: eine vergrößerte Darstellung des Details II gemäß Fig. 1 in der Draufsicht.
- Fig. 3: eine vergrößerte Darstellung des Details II gemäß Fig. 1 einer bevorzugten Ausführungsform der Erfindung in der Draufsicht.
- Fig. 4: eine vergrößerte Darstellung des Details II gemäß Fig. 1 einer zweiten bevorzugten Ausführungsform der Erfindung in der Draufsicht.
- Fig. 5: eine vergrößerte Darstellung des Details II gemäß Fig. 1 einer dritten bevorzugten Ausführungsform der Erfindung in der Draufsicht.
- Fig. 6: eine vergrößerte Darstellung des Details II gemäß Fig. 1 einer vierten bevorzugten Ausführungsform der Erfindung in der Draufsicht.

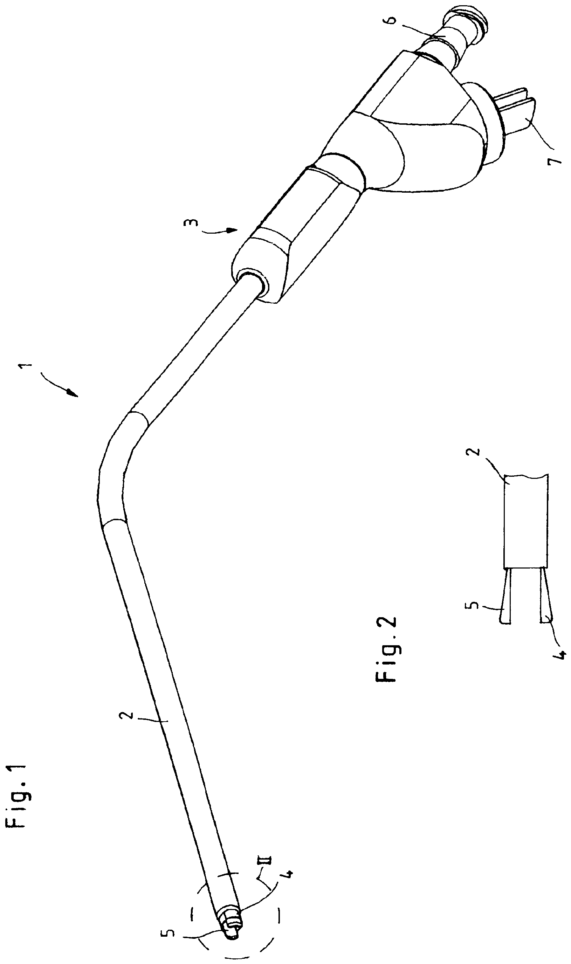

Fig. 1 zeigt ein als bipolares Koagulationsinstrument 1 ausgebildetes medizinisches Koagulationsinstrument 1, das zusätzlich zur Ausbildung als Koagulationsinstrument auch als Sauginstrument ausgebildet ist.

Dieses dargestellte medizinische bipolaren Koagulationsinstrument 1 besteht im Wesentlichen aus einem hohlen, als Saug-/Spülkanal ausgebildeten Schaft 2, einer am proximalen Ende des Schaftes 2 angeordneten Handhabe 3, sowie zwei den hohlen Schaft 2 distalseitig überragenden Elektrodenspitzen 4 und 5.

Zur Ausbildung des hohlen Schaftes 2 als Saug- und/oder Spülkanal weist die Handhabe 3 an ihrem proximalen Ende einen Saug- und/oder Spülanschluss 6 zur Ankopplung einer externen Saug- und/oder Spülleitung auf. Die Beaufschlagung der Elektrodenspitzen 4 und 5 erfolgt über einen an der Handhabe 3 angeordneten Stromanschluss 7.

Wie insbesondere der Detailansicht gemäß Fig. 2 zu entnehmen ist, sind die fingerartig vorspringenden Elektrodenspitzen 3 und 4 einander gegenüberliegend und gegeneinander elektrisch isoliert am distalen Ende des Schaftes 2 so angeordnet, dass sie das distale Ende des Schaftes 2 überragen.

Um den Elektrodenspitzen 4 und 5 eine höhere Standfestigkeit zu verleihen sind die Elektrodenspitzen 4 und 5 sich nach distal aufweitend ausgebildet. Bei der dargestellten Ausführungsform sind die Elektrodenspitzen 4 und 5 sich nach distal konisch erweiternd so ausgebildet, dass die Elektrodenspitzen 4 und 5 am freien distalen Ende deutlich dicker ausgebildet sind als am proximalen Übergang zum Schaft 2.

Alternativ zu der dargestellten konischen Erweiterung der Elektrodenspitzen 4 und 5 hin zum distalen Ende ist es auch möglich, diese distalseitige Erweiterung der Elektrodenspitzen 4 und 5 durch eine fächerförmige Erweiterung der freien distalen Enden der Elektrodenspitzen 4 und 5 zu erzielen.

Zusätzlich zur Erhöhung der Standfestigkeit der Elektrodenspitzen 4 und 5 bewirkt das Aufweiten der freien distalen Enden der Elektrodenspitzen 4 und 5 eine Vergrößerung Koagulationsflächen, also der Flächen, über die der Strom unter Hitzeentwicklung in das Patientengewebe eintritt.

Ein weiterer entscheidender Vorteil der Aufweitung der distalseitigen freien Enden der elektrodenspitzen 4 und 5 besteht darin, dass die Gefahr, den Patienten beim Einführen des Koagulationsinstruments 1 in das Operationsgebiet zu verletzen deutlich reduziert wird, wenn die freien Enden der Elektrodenspitzen 4 und 5 nicht mehr dünn und schmal, sondern erweitert und verdickt ausgebildet sind.

Um die Möglichkeit der atraumatischen Verwendung der Koagulationsinstrumente 1 über die Aufweitung der Elektrodenspitzen 4 und 5 hinaus weiter zu verbessern, sind die freien Enden der in Fig. 2 dargestellten Elektrodenspitzen 4 und 5 zusätzlich abgerundet ausgebildet.

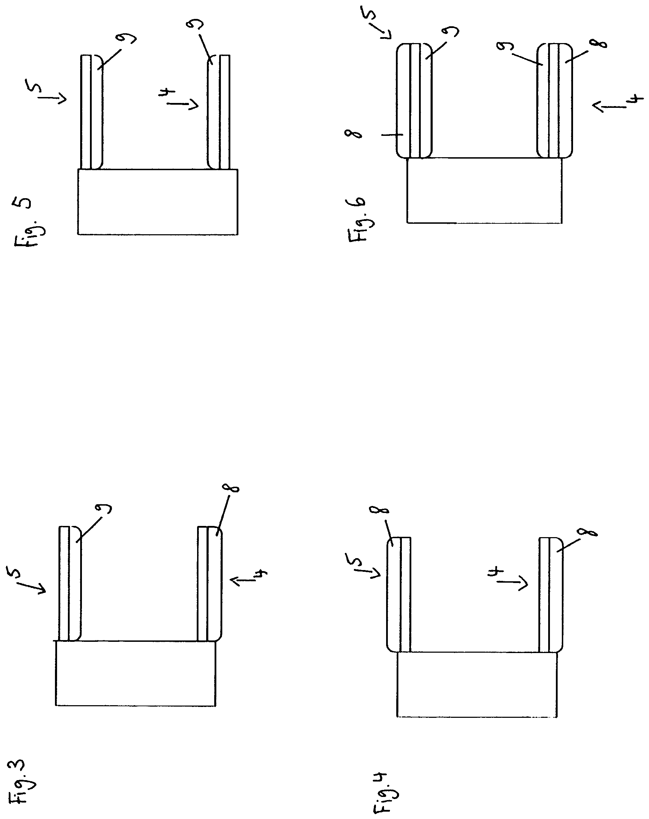

Fig. 3 zeigt eine bevorzugte Ausführungsform der Erfindung in vergrößerter Darstellung, also entsprechend Detail II gemäß Fig. 1 in der Draufsicht.

Hier erweitern sich die Elektrodenspitzen 4 und 5 nach distal, und zwar gewissermaßen stufenförmig. Und zwar indem auf die Elektrodenspitzen 4 und 5 Plättchen 8, 9 aufgebracht, insbesondere aufgeschweißt sind. Denkbar sind aber auch alle Fügearten und Kombination für die Plättchen 8 und 9 an die jeweilige Elektrodenspitze 4 beziehungsweise 5.

Bei dieser bevorzugten Ausführungsform ist auf die Elektrodenspitze 4 ein Plättchen 8 außen aufgebracht. Das Plättchen 8 kann wie hier dargestellt eine konstante Materialstärke haben.

Die Materialstärke des Plättchens 8 könnte sich alternativ aber auch in Achsrichtung des Koagulationsinstruments 1 ändern, insbesondere nach distal erweitern, insbesondere konisch. Ebenso kann sich die Materialstärke des Plättchens 8 nach distal verringern, insbesondere konisch. Außer konisch kann die Änderung der Materialstärke des Plättchens 8 auch in jeder anderen Art ausgeführt sein, beispielsweise konvex oder konkav, stetig oder unstetig. Besonders bevorzugt ist, dass das Plättchen 8 an seinen Kanten, insbesondere der distalen Kante abgegratet, insbesondere abgerundet ist.

Dieses Plättchen 8 hat vorzugsweise die Form und insbesondere die Breite der Elektrodenspitze 4, so dass der Gesamtaufbau Elektrodenspitze 4 mit daran aufgebrachtem Plättchen 8 eine verdickte Elektrodenspitze 4 bilden. Dabei ist wichtig, dass das Plättchen 8 im Wesentlichen bis zur distalen Endfläche der Elektrode beziehungsweise der Elektrodenspitze 4 reicht.

Obwohl in der Fig. 3 nur im Schnitt dargestellt, ist das Plättchen vorzugsweise ein Rohrsegment, insbesondere jedoch von der Form und Wölbung der Elektrodenspitze 4.

Auf die Elektrodenspitze 5 gemäß Fig. 3 ist ein Plättchen 9 innen aufgebracht.
Wie Plättchen 8 kann Plättchen 9 eine konstante Materialstärke haben. Alternativ könnte Plättchen 9 eine sich wie für Plättchen 8 beschrieben ändernde Materialstärke aufweisen. Dabei sind alle Kombination von sich ändernden Materialstärken bei Plättchen 8 und Plättchen 9 denkbar.

Form und insbesondere die Breite von Plättchen 9 können wie bei Plättchen 8 sein, wobei alle Kombinationen dieser verschiedenen Arten der Form beziehungsweise Breite der Plättchen 8 und 9 denkbar sind.

Fig. 4 zeigt eine zweite bevorzugte Ausführungsform der Erfindung in vergrößerter Darstellung, also entsprechend Detail II gemäß Fig. 1 in der Draufsicht.

Wie in Fig. 3 erweitern sich hier die Elektrodenspitzen 4 und 5 nach distal, und zwar gewissermaßen stufenförmig. Und zwar indem auf die Elektrodenspitzen 4 und 5 Plättchen 8 aufgebracht, insbesondere aufgeschweißt sind. Denkbar sind aber auch alle Fügearten und -kombinationen für die Plättchen 8 an die jeweilige Elektrodenspitze 4 beziehungsweise 5.

Bei dieser zweiten bevorzugten Ausführungsform ist auf die Elektrodenspitze 4 ein Plättchen 8 außen aufgebracht. Das Plättchen 8 kann wie hier dargestellt eine konstante Materialstärke haben.

Die Materialstärke des Plättchens 8 könnte sich alternativ aber auch in Achsrichtung des Koagulationsinstruments 1 ändern, insbesondere nach distal erweitern, insbesondere konisch. Ebenso kann sich die Materialstärke des Plättchens 8 nach distal verringern, insbesondere konisch. Außer konisch kann die Änderung der Materialstärke des Plättchens 8 auch in jeder anderen Art ausgeführt sein, beispielsweise konvex oder konkav, stetig oder unstetig. Besonders bevorzugt ist, dass das Plättchen 8 an seinen Kanten, insbesondere der distalen Kante abgegratet, insbesondere abgerundet ist.

Dieses Plättchen 8 hat vorzugsweise die Form und insbesondere die Breite der Elektrodenspitze 4, so dass der Gesamtaufbau Elektrodenspitze 4 mit daran aufgebrachtem Plättchen 8 eine verdickte Elektrodenspitze 4 bilden. Dabei ist wichtig, dass das Plättchen 8 im Wesentlichen bis zur distalen Endfläche der Elektrode beziehungsweise der Elektrodenspitze 4 reicht.

Obwohl in der Fig. 4 nur im Schnitt dargestellt, ist das Plättchen vorzugsweise ein Rohrsegment, insbesondere jedoch von der Form und Wölbung der Elektrodenspitze 4.

Auf die Elektrodenspitze 5 gemäß Fig. 4 ist ein Plättchen 8 ebenso außen aufgebracht.

Dieses Plättchen 8 kann eine konstante Materialstärke haben oder auch wie für Plättchen 8 auf der Elektrodenspitze 4 beschrieben eine sich ändernde Materialstärke aufweisen. Dabei sind alle Kombination von sich ändernden Materialstärken der beiden Plättchen 8 denkbar.

Die Form und insbesondere die Breite dieses Plättchens 8 können wie Plättchen 8 auf der Elektrodenspitze 4 beschrieben sein, wobei alle Kombinationen dieser verschiedenen Arten der Form beziehungsweise Breite der beiden Plättchen 8 denkbar sind.

Fig. 5 zeigt eine dritte bevorzugte Ausführungsform der Erfindung in vergrößerter Darstellung, also entsprechend Detail II gemäß Fig. 1 in der Draufsicht.

Wie in Fig. 3 und 4 erweitern sich hier die Elektrodenspitzen 4 und 5 nach distal, und zwar gewissermaßen stufenförmig. Und zwar indem auf die Elektrodenspitzen 4 und 5 Plättchen 9 aufgebracht, insbesondere aufgeschweißt sind. Denkbar sind aber auch alle Fügearten und -kombinationen für die Plättchen 9 an die jeweilige Elektrodenspitze 4 beziehungsweise 5.

Bei dieser dritten bevorzugten Ausführungsform ist auf die Elektrodenspitze 4 ein Plättchen 9 innen aufgebracht. Das Plättchen 9 kann wie hier dargestellt eine konstante Materialstärke haben.

Die Materialstärke des Plättchens 9 könnte sich alternativ aber auch in Achsrichtung des Koagulationsinstruments 1 ändern, insbesondere nach distal erweitern, insbesondere konisch. Ebenso kann sich die Materialstärke des Plättchens 9 nach distal verringern, insbesondere konisch. Außer konisch kann die Änderung der Materialstärke des Plättchens 9 auch in jeder anderen Art ausgeführt sein, beispielsweise konvex oder konkav, stetig oder unstetig. Besonders bevorzugt ist, dass das Plättchen 9 an seinen Kanten, insbesondere der distalen Kante abgegratet, insbesondere abgerundet ist.

Dieses Plättchen 9 hat vorzugsweise die Form und insbesondere die Breite der Elektrodenspitze 4, so dass der Gesamtaufbau Elektrodenspitze 4 mit daran aufgebrachtem Plättchen 9 eine verdickte Elektrodenspitze 4 bilden. Dabei ist wichtig, dass das Plättchen 9 im Wesentlichen bis zur distalen Endfläche der Elektrode beziehungsweise der Elektrodenspitze 4 reicht.

Obwohl in der Fig. 5 nur im Schnitt dargestellt, ist das Plättchen vorzugsweise ein Rohrsegment, insbesondere jedoch von der Form und Wölbung der Elektrodenspitze 4.

Auf die Elektrodenspitze 5 gemäß Fig. 5 ist ein Plättchen 9 ebenso innen aufgebracht.

Dieses Plättchen 9 kann eine konstante Materialstärke haben oder auch wie für Plättchen 9 auf der Elektrodenspitze 4 beschrieben eine sich ändernde Materialstärke aufweisen. Dabei sind alle Kombination von sich ändernden Materialstärken der beiden Plättchen 9 denkbar.

Die Form und insbesondere die Breite dieses Plättchens 9 können wie Plättchen 9 auf der Elektrodenspitze 4 beschrieben sein, wobei alle Kombinationen dieser verschiedenen Arten der Form beziehungsweise Breite der beiden Plättchen 9 denkbar sind.

Fig. 6 zeigt eine vierte bevorzugte Ausführungsform der Erfindung in vergrößerter Darstellung, also entsprechend Detail II gemäß Fig. 1 in der Draufsicht.

Wie in Fig. 3 und 4 erweitern sich hier die Elektrodenspitzen 4 und 5 nach distal, und zwar gewissermaßen stufenförmig. Und zwar indem auf die Elektrodenspitzen 4 und 5 jeweils Plättchen 8 und 9 aufgebracht, insbesondere aufgeschweißt sind. Denkbar sind aber auch alle Fügearten und -kombinationen für die Plättchen 8 und 9 an die jeweilige Elektrodenspitze 4 beziehungsweise 5.

Bei dieser vierten bevorzugten Ausführungsform sind auf die Elektrodenspitze 4 ein Plättchen 8 innen sowie ein Plättchen 9 außen aufgebracht. Das Plättchen 8 wie das Plättchen 9 kann wie hier dargestellt eine konstante Materialstärke haben.

Die Materialstärke der Plättchens 8 beziehungsweise 9 könnte sich alternativ aber jeweils auch in Achsrichtung des Koagulationsinstruments 1 ändern, insbesondere nach distal erweitern, insbesondere konisch. Ebenso kann die Materialstärke der Plättchen 8 beziehungsweise 9 sich nach distal verringern, insbesondere konisch. Außer konisch kann die Änderung der Materialstärke der Plättchens 8 beziehungsweise 9 auch in jeder anderen Art ausgeführt sein, beispielsweise konvex oder konkav, stetig oder unstetig. Besonders bevorzugt ist, dass die Plättchen 8 beziehungsweise 9 an ihren Kanten, insbesondere der distalen Kante abgegratet, insbesondere abgerundet ist. Dabei können sich die Materialstärken und Abgratungen/-rundungen der beiden Plättchen 8 und 9 auch unterschiedlich verhalten beziehungsweise bei einem der Plättchen 8 und 9 könnte sich die Materialstärke ändern und beim anderen nicht, so dass alle entsprechenden Kombinationen denkbar sind.

Das Plättchen 8 beziehungsweise das Plättchen 9 haben vorzugsweise die Form und insbesondere die Breite der Elektrodenspitze 4, so dass der Gesamtaufbau Elektrodenspitze 4 mit daran aufgebrachten Plättchen 8 und 9 eine verdickte Elektrodenspitze 4 bilden. Dabei ist wichtig, dass das Plättchen 8 und das Plättchen 9 im Wesentlichen bis zur distalen Endfläche der Elektrode beziehungsweise der Elektrodenspitze 4 reichen.

Obwohl in der Fig. 6 nur im Schnitt dargestellt, sind die Plättchen 8 und 9 vorzugsweise Rohrsegmente, insbesondere jedoch von der Form und Wölbung der Elektrodenspitze 4.

Auf die Elektrodenspitze 5 gemäß Fig. 6 sind ein Plättchen 8 innen sowie ein Plättchen 9 außen aufgebracht.

Diese Plättchen 8 beziehungsweise 9 können jeweils eine konstante Materialstärke haben oder auch wie für die Plättchen 8 beziehungsweise 9 auf der Elektrodenspitze 4 beschrieben eine sich ändernde Materialstärke aufweisen. Dabei sind alle Kombination von sich ändernden Materialstärken der beiden Plättchen 8 beziehungsweise 9 denkbar.

Die Form und insbesondere die Breite dieser Plättchens 8 beziehungsweise 9 können wie für die Plättchen 8 beziehungsweise 9 auf der Elektrodenspitze 4 beschrieben sein, wobei alle Kombinationen dieser verschiedenen Arten der Form beziehungsweise Breite der beiden Plättchen 8 beziehungsweise 9 denkbar sind.

In gemäß der Erfindung beziehungsweise den dargelegten Ausführungsformen ausgebildetes medizinisches Koagulationsinstrument zeichnet sich dadurch aus, dass aufgrund der distalen Aufweitung der freien Enden der Elektrodenspitzen 4 und 5 eine atraumatische Handhabung des Koagulationsinstruments bei gleichzeitig deutlich verbesserter Standfestigkeit der Elektrodenspitzen 4 und 5 gewährleistet ist.

Alternativ zu der Darstellung in den Abbildungen Fig. 1, 2, 3, 4, 5 und 6 ist es selbstverständlich auch möglich, diese distale Aufweitung der Elektrodenspitzen 4 und 5 auch bei anderen bipolaren Koagulationsinstrumenten oder auch bei den mit nur einer Elektrodenspitze 4 ausgestatteten monopolaren Koagulationsinstrumenten einzusetzen.

### Bezugszeichenliste

- 1: medizinisches Koagulationsinstrument
- 2: Schaft
- 3: Handhabe
- 4: Elektrode/Elektrodenspitze
- 5: Elektrode/Elektrodenspitze
- 6: Saug- und/oder Spülanschluss
- 7: Stromanschluss
- 8: Plättchen, außen
- 9: Plättchen, innen

## Patentansprüche

1. Medizinisches Koagulationsinstrument mit einem Schaft (2) sowie mit mindestens einer das distale Ende des Schaftes (2) überragenden Elektrodenspitze (4),
wobei die mindestens eine Elektrodenspitze (4) nach distal aufgeweitet oder sich aufweitend ausgebildet ist, und die Elektrodenspitze (4, 5) sich nach distal verdickend ausgebildet ist, **dadurch gekennzeichnet, dass** auf die Innenseite und/oder die Außenseite der mindestens einen Elektrodenspitze (4, 5) ein Plättchen (8, 9) aufgebracht ist und das distale Ende des Plättchens (8, 9) im Wesentlichen bis zur distalen Endfläche der Elektrodenspitze (4, 5) reicht, um die verdickte Elektrodenspitze (4, 5) zu bilden, so dass das Aufweiten des distalen Endes der Elektrodenspitze (4, 5) eine Vergrößerung der Koagulationsfläche bewirkt.

2. Medizinisches Koagulationsinstrument nach Anspruch 1, **dadurch gekennzeichnet, dass** am distalen Ende des Schaftes (2) zwei das distale Ende des Schaftes (2) überragende, gegeneinander elektrisch isolierten Elektrodenspitzen (4, 5) angeordnet sind, die beide nach distal aufgeweitet oder sich aufweitend ausgebildet ist.

3. Medizinisches Koagulationsinstrument nach einem der Ansprüche 1 bis 3, **dadurch gekennzeichnet, dass** die Elektrodenspitze (4, 5) oder Elektrodenspitzen (4, 5) sich nach distal konisch erweiternd ausgebildet sind.

4. Medizinisches Koagulationsinstrument nach einem der Ansprüche 1 bis 4, **dadurch gekennzeichnet, dass** die Elektrodenspitze (4, 5) oder Elektrodenspitzen (4, 5) sich nach distal fächerförmig erweiternd ausgebildet sind.

5. Medizinisches Koagulationsinstrument nach einem der Ansprüche 1 bis 5, **dadurch gekennzeichnet, dass** die Elektrodenspitze (4, 5) oder Elektrodenspitzen (4, 5) sich nach distal stufenförmig erweiternd ausgebildet sind.

6. Medizinisches Koagulationsinstrument nach Anspruch 6, **dadurch gekennzeichnet, dass** die Elektrodenspitze (4, 5) nach distal Abschnitte konstanter Materialstärke aufweist.

7. Medizinisches Koagulationsinstrument nach einem der Ansprüche 1 bis 9, **dadurch gekennzeichnet, dass** das Plättchen (8, 9) eine konstante Materialstärke aufweist.

8. Medizinisches Koagulationsinstrument nach einem der Ansprüche 1 bis 9, **dadurch gekennzeichnet, dass** sich die Materialstärke des Plättchens (8, 9) nach distal ändert, insbesondere vergrößert.

9. Medizinisches Koagulationsinstrument nach einem der Ansprüche 1 bis 9, **dadurch gekennzeichnet, dass** sich die Materialstärke des Plättchens (8, 9) in tangentialer Richtung ändert, insbesondere nach außen vergrößert.

10. Medizinisches Koagulationsinstrument nach einem der Ansprüche 1 bis 12, **dadurch gekennzeichnet, dass** die Materialstärke des Plättchens (8, 9) im Wesentlichen gleich der Materialstärke der Elektrodenspitze (4, 5) ist.

11. Medizinisches Koagulationsinstrument nach einem der Ansprüche 1 bis 13, **dadurch gekennzeichnet, dass** die freien Enden der Elektrodenspitzen (4, 5) abgerundet ausgebildet sind.

## Claims

1. Medical coagulation instrument with a shank (2) and with at least one electrode tip (4) protruding beyond the distal end of the shank (2), the at least one electrode tip (4) being designed widened or widening in the distal direction, and the electrode tip (4, 5) becoming thicker in the distal direction, **characterized in that** a small plate (8, 9) is mounted on the inner face and/or the outer face of the at least one electrode tip (4, 5), and the distal end of the small plate (8, 9) reaches substantially as far as the distal end surface of the electrode tip (4, 5) in order to form the thickened electrode tip (4, 5), such that the widening of the distal end of the electrode tip (4, 5) brings about an increase in the size of the coagulation surface.

2. Medical coagulation instrument according to Claim 1, **characterized in that** two mutually electrically insulated electrode tips (4, 5) arranged at the distal end of the shank (2) protrude beyond the distal end of the shank (2) and are both designed widened or widening in the distal direction.

3. Medical coagulation instrument according to one of Claims 1 to 3, **characterized in that** the electrode tip (4, 5) or electrode tips (4, 5) are designed widening conically in the distal direction.

4. Medical coagulation instrument according to one of Claims 1 to 4, **characterized in that** the electrode tip (4, 5) or electrode tips (4, 5) are designed widening in a fan shape in the distal direction.

5. Medical coagulation instrument according to one of Claims 1 to 5, **characterized in that** the electrode tip (4, 5) or electrode tips (4, 5) are designed widening in a step shape in the distal direction.

6. Medical coagulation instrument according to Claim 6, **characterized in that** the electrode tip (4, 5) has portions of constant material thickness in the distal direction.

7. Medical coagulation instrument according to one of Claims 1 to 9, **characterized in that** the small plate (8, 9) has a constant material thickness.

8. Medical coagulation instrument according to one of Claims 1 to 9, **characterized in that** the material thickness of the small plate (8, 9) changes in the distal direction, in particular increases in the distal direction.

9. Medical coagulation instrument according to one of Claims 1 to 9, **characterized in that** the material thickness of the small plate (8, 9) changes in the tangential direction, in particular increases in the outward direction.

10. Medical coagulation instrument according to one of Claims 1 to 12, **characterized in that** the material thickness of the small plate (8, 9) is substantially equal to the material thickness of the electrode tip (4, 5).

11. Medical coagulation instrument according to one of Claims 1 to 13, **characterized in that** the free ends of the electrode tips (4, 5) have a rounded shape.

## Revendications

1. Instrument de coagulation médical avec un bras (2) ainsi qu'avec au moins une pointe d'électrode (4) dépassant l'extrémité distale du bras (2), dans lequel ladite au moins une pointe d'électrode (4) est évasée ou s'évase en direction distale et la pointe d'électrode (4, 5) s'épaissit en direction distale, **caractérisé en ce qu'**une plaquette (8, 9) est montée sur le côté intérieur et/ou le côté extérieur de ladite au moins une pointe d'électrode (4, 5) et l'extrémité distale de la plaquette (8, 9) s'étend essentiellement jusqu'à la face d'extrémité distale de la pointe d'électrode (4, 5), pour former la pointe d'électrode épaissie (4, 5), de telle manière que l'évasement de l'extrémité distale de la pointe d'électrode (4, 5) entraîne un agrandissement de la face de coagulation.

2. Instrument de coagulation médical selon la revendication 1, **caractérisé en ce que** deux pointes d'électrode (4, 5) électriquement isolées l'une de l'autre et dépassant l'extrémité distale du bras (2) sont disposées à l'extrémité distale du bras (2), et toutes les deux sont évasées ou s'évasent en direction distale.

3. Instrument de coagulation médical selon l'une quelconque des revendications 1 à 3, **caractérisé en ce que** la pointe d'électrode (4, 5) ou les pointes d'électrode (4, 5) sont évasées en forme de cône en direction distale.

4. Instrument de coagulation médical selon l'une quelconque des revendications 1 à 4, **caractérisé en ce que** la pointe d'électrode (4, 5) ou les pointes d'électrode (4, 5) sont évasées en forme d'éventail en direction distale.

5. Instrument de coagulation médical selon l'une quelconque des revendications 1 à 5, **caractérisé en ce que** la pointe d'électrode (4, 5) ou les pointes d'électrode (4, 5) s'élargissent par degrés en direction distale.

6. Instrument de coagulation médical selon la revendication 6, **caractérisé en ce que** la pointe d'électrode (4, 5) présente des sections d'épaisseur de matière constante en direction distale.

7. Instrument de coagulation médical selon l'une quelconque des revendications 1 à 9, **caractérisé en ce que** la plaquette (8, 9) présente une épaisseur de matière constante.

8. Instrument de coagulation médical selon l'une quelconque des revendications 1 à 9, **caractérisé en ce que** l'épaisseur de matière de la plaquette (8, 9) varie en direction distale, en particulier augmente.

9. Instrument de coagulation médical selon l'une quelconque des revendications 1 à 9, **caractérisé en ce que** l'épaisseur de matière de la plaquette (8, 9) varie en direction tangentielle, en particulier augmente vers l'extérieur.

10. Instrument de coagulation médical selon l'une quelconque des revendications 1 à 12, **caractérisé en ce que** l'épaisseur de matière de la plaquette (8, 9) est essentiellement égale à l'épaisseur de matière de la pointe d'électrode (4, 5).

11. Instrument de coagulation médical selon l'une quelconque des revendications 1 à 13, **caractérisé en ce que** les extrémités libres des pointes d'électrode (4, 5) sont de forme arrondie.
